# EUROPÄISCHE PATENTSCHRIFT

(11) **EP 2 424 600 B1**
(45) Veröffentlichungstag und Bekanntmachung des Hinweises auf die Patenterteilung: **01.10.2014**
(21) Anmeldenummer: 10722558.3
(22) Anmeldetag: 27.04.2010
(51) Int. Cl.: A61M 5/34, A61M 39/10

(54) **LUER-LOCK-ANSCHLUSS**
LUER LOCK CONNECTION
RACCORD LUER LOCK

(30) Priorität: 30.04.2009 DE 102009019340
(43) Veröffentlichungstag der Anmeldung: 07.03.2012
(73) Patentinhaber: Transcoject GmbH, 24539 Neumünster (DE)
(72) Erfinder: HEINZ, Jochen, 24220 Flintbek (DE); SCHILLING, Dieter, 24613 Aukrug-Innien (DE); LUCYGA, Ralf, 24241 Schierensee (DE)
(74) Vertreter: Patentanwälte Vollmann & Hemmer
(86) Internationale Anmeldenummer: PCT/DE2010/000478
(87) Internationale Veröffentlichungsnummer: WO 2010/124676

(56) Entgegenhaltungen:
- EP-A1- 0 869 826
- DE-U1-202004 012 714
- FR-A1- 2 809 316
- US-A- 5 047 021
- US-A- 5 984 373

## Beschreibung

Die Erfindung betrifft einen Luer-Lock-Anschluss.

Es sind Luer-Lock-Anschlüsse bekannt, bei welchen die den Luer-Konus umgebende Gewindehülse relativ zu dem Luer-Konus um dessen Längsachse drehbar ist. Diese Ausgestaltung kann beabsichtigt sein und wird z. B. dazu benutzt, durch Drehung der Gewindehülse ein weibliches Gegenstück auf dem Luer-Konus fixieren zu können, ohne letzteres verdrehen zu müssen. Eine Drehung der Gewindehülse kann jedoch auch unvermeidlich sein, wenn zum Beispiel Luer-Konus und Gewindehülse aus unterschiedlichen Materialien bestehen und aufeinander gesteckt sind, wie es z. B. bei Glasspritzen üblich ist. Problematisch bei dieser Ausgestaltung ist, dass sich die Verbindung leicht lösen kann, insbesondere, wenn Feuchtigkeit oder Schmiermittel wie z. B. Silikonöl auf den Luer-Konus oder in das Gewinde der Gewindehülse gelangt, oder besonders glatte Materialien wie z. B. PTFE verwendet werden.

EP 0 869 826 offenbart einen Luer-Lock-Verbinder, bei welchem eine drehbare Gewindehülse über eine konische Klemmfläche beim Festziehen des Gewindes axial geklemmt wird. Ähnliche Ausgestaltungen sind aus US 5,984,373, DE 20 2004 012714 U1 sowie US 5,047,021 bekannt. Nachteilig bei diesen Ausgestaltungen ist, dass die Gewindehülse ein gewisses axiales Spiel aufweisen muss, sodass zum Festziehen durch Drehung der Gewindehülse diese um ein größeres Maß gedreht werden muss. FR 2 809 316 offenbart Reibmittel auf der kreisringförmigen Anlagefläche zwischen Gewindehülse und Luer-Konus vorzusehen. Dadurch erschwert sich die Drehung der Gewindehülse, sodass das Festziehen der Luer-Lock-Verbindung erschwert wird.

Im Hinblick darauf ist es Aufgabe der Erfindung, einen Luer-Lock-Anschluss mit einer Gewindehülse, welche nicht fest mit den Luer-Konus verbunden ist, dahingehend zu verbessern, dass die Verbindung dieses Luer-Lock-Anschlusses mit einem weiblichen Gegenstück verbessert gegen unbeabsichtigtes Lösen gesichert ist.

Diese Aufgabe wird durch einen Luer-Lock-Anschluss mit den in Anspruch 1 angegebenen Merkmalen gelöst. Bevorzugte Ausführungsformen ergeben sich aus den Unteransprüchen der nachfolgenden Beschreibung, sowie den beigefügten Figuren.

Der erfindungsgemäße Luer-Lock-Anschluss weist bekannterweise einen Luer-Konus und eine diesen umgebende Gewindehülse auf. Dabei ist die Gewindehülse an dem dem freien Ende des Luer-Konus abgewandten Ende bzw. Fuß des Luer-Konus angebracht. Dazu kann die Gewindehülse einen radialen nach innen gerichteten Vorsprung aufweisen, welcher in eine ringförmige Nut am Außenumfang des Fußes des Luer-Konus eingreift. So ist die Gewindehülse um den Luer-Konus um dessen Längsachse drehbar. Dabei kann beispielsweise der radial nach innen gerichtete Vorsprung der Gewindehülse in der Nut in Umfangsrichtung gleiten. Nut und Vorsprung bilden dabei eine axiale Sicherung, welche die axiale Beweglichkeit der Gewindehülse in Richtung der Längsachse des Luer-Konus begrenzt bzw. verhindert.

Erfindungsgemäß sind zwischen der Gewindehülse und dem Fuß des Luer-Konus zusätzliche Klemmmittel vorgesehen, welche eine Verdrehsicherung bilden, wenn ein weibliches Gegenstück auf den Luer-Konus aufgesetzt bzw. in die Gewindehülse eingeschraubt ist. Durch diese Klemmmittel wird dann verhindert, dass die Gewindehülse sich unbeabsichtigt, insbesondere selbsttätig, beispielsweise durch äußere Erschütterungen, verdrehen kann und so zu einem Lösen der Verbindung zwischen dem Luer-Lock-Anschluss und dem weiblichen Gegenstück führen würde. Die Klemmmittel sind dazu derart ausgebildet, dass sie, wenn auf den Luer-Konus ein weibliches Gegenstück aufgesetzt ist, durch Drehung der Gewindehülse oder Drehung des weiblichen Gegenstückes in der Gewindehülse eine Klemmung zwischen der Gewindehülse und dem Fuß des Luer-Konus erzielen. Durch diese Klemmung wird eine zusätzliche Sicherung der Verbindung erreicht. Bei herkömmlichen Luer-Lock-Anschlüssen erfolgt eine reibungsbedingte Sicherung zwischen der konischen Außenfläche des Luer-Konus und dem weiblichen Gegenstück. Zusätzlich wird durch das Gewinde der Gewindehülse eine axiale Sicherung erreicht. Bei herkömmlichen Luer-Lock-Anschlüssen wir die Gewindehülse jedoch lediglich durch die Reibung im Gewinde gehalten. Erfindungsgemäß wird nun eine zusätzliche Reib- und/oder Formschlussverbindung bzw. Klemmung durch die Klemmmittel direkt zwischen der Gewindehülse und deren Lagerung am Fuß des Luer-Konus geschaffen. Die Klemmmittel sind, vorzugsweise direkt an der Gewindehülse und/oder am Fuß des Luer-Konus, so ausgebildet, dass zwischen der Gewindehülse und dem Fuß des Luer-Konus zur zusätzlichen Sicherung ein form- und kraftschlüssiger Eingriff zur Verdrehsicherung erfolgt. D. h. die Klemmmittel bilden form- und kraftschlüssig wirkende Eingriffselemente. Durch diese zusätzliche Sicherung kann ein sicherer Halt geschaffen werden, insbesondere auch dann, wenn Feuchtigkeit oder Schmiermittel auf das Gewinde oder den Luer-Konus gelangen sollte. Da die erfindungsgemäßen Klemmmittel darüber hinaus im Fußbereich des Luer-Konus, d.h. an dessen freien Ende des Luer-Konus abgewandten Ende angeordnet sind, kann verhindert werden, dass Feuchtigkeit, welche aus dem Luer-Lock-Anschluss, beispielsweise bei vorgefüllten Kartuschen, austritt, in den Bereich der Klemmmittel gelangt.

Die Klemmmittel weisen zumindest eine Anlagefläche am Fuß des Luer-Konus und eine Gegen-Anlagefläche an der Gewindehülse auf, welche derart angeordnet sind, dass sie bei auf den Luer-Konus aufgesetztem weiblichen Gegenstück durch relative Drehung zwischen der Gewindehülse und dem weiblichen Gegenstück mit einander in Eingriff treten. Die relative Drehung zwischen der Gewindehülse und dem weiblichen Gegenstück kann durch Drehung der Gewindehülse und/oder des weiblichen Gegenstückes erfolgen. Solange das weibliche Gegenstück nicht aufgesetzt ist, ist die Gewindehülse vorzugsweise frei drehbar. So kann die Gewindehülse in bekannter Weise auf das Gewinde eines weiblichen Gegenstückes aufgeschraubt werden und so das weibliche Gegenstück über die Gewindehülse auf den Luer-Konus gezogen werden. Anlagefläche und Gegen-Anlagefläche sind so angeordnet, bzw. ausgestaltet, dass sie erst dann in reibenden Kontakt bzw. in Eingriff treten, wenn das weibliche Gegenstück auf dem Luer-Konus geklemmt wird. Auf diese Weise wird eine freie Drehbarkeit der Gewindehülse bis zur festen Anlage des weiblichen Gegenstückes auf den Luer-Konus erreicht und erst dann die zusätzliche Klemmung zwischen Anlagefläche und Gegen-Anlagefläche als Verdrehsicherung der Gewindehülse erreicht.

Die Anlagefläche und/oder die Gegen-Anlagefläche sind ringförmig, insbesondere kreisringförmig ausgebildet. Dabei kann die Gegen-Anlagefläche die Anlagefläche ringförmig umgreifen. Anlagefläche und/oder Gegen-Anlagefläche können dabei Teile der Führung der Gewindehülse am Fuß des Luer-Konus sein. Das heißt die Anlagefläche kann Teil einer Nut am Fuß des Luer-Konus sein, beispielsweise der Nutboden oder eine der Nutwandungen. Die Gegen-Anlagefläche kann dabei eine entsprechend ringförmige Innenfläche oder eine der axialen Stirnseiten eines radial nach innen gerichteten Vorsprunges der Gewindehülse sein, welcher in die zuvor beschriebene Nut eingreift. Durch die ringförmige Ausgestaltung von Anlagefläche und/oder Gegen-Anlagefläche kann erreicht werden, dass eine Klemmung in jeder beliebigen Winkelposition zwischen Luer-Konus und Gewindehülse erreicht werden kann. Dies ist insbesondere deshalb von Vorteil, da üblicherweise das weibliche Gegenstück auf den Luer-Konus auch in beliebiger Winkelposition bezüglich der Längsachse des Luer-Konus aufgesetzt werden kann.

Die Gegen-Anlagefläche liegt der Anlagefläche radial bezüglich der Längsachse des Luer-Konus gegenüber. So kann die Anlagefläche eine nach außen gewandte zylindrische Ringfläche sein, welche sich um den Fuß des Luer-Konus, beispielsweise in einer Nut zur Aufnahme eines radialen Vorsprungs der Gewindehülse erstreckt. Die Gegen-Anlagefläche kann eine ringförmige zylindrische nach innen gewandte Fläche an der Gewindehülse sein, welche so die Anlagefläche umfänglich umgibt. Auf diese Weise kann sichergestellt werden, dass in jeder Winkelposition die Anlagefläche und die Gegen-Anlagefläche einander gegenüberliegen. Es ist jedoch auch denkbar, dass die Anlagefläche oder die Gegen-Anlagefläche sich nicht über den vollen Umfang um die Längsachse des Luer-Konus erstrecken, sondern lediglich über begrenzte Umfangsabschnitte. Dabei ist dann vorzugsweise die jeweils gegenüberliegende andere Fläche als geschlossener Ring ausgebildet, sodass weiterhin sichergestellt ist, dass in jeder Winkelposition eine Anlage erreicht werden kann.

Ferner weist die Gegen-Anlagefläche bezüglich der Längsachse des Luer-Konus einen Abschnitt auf, welcher radial weiter innen gelegen ist, als ein Abschnitt der Anlagefläche, wobei die Gewindehülse am Fuß des Luer-Konus mit radialem Spiel angeordnet ist. Dabei ist das radiale Spiel zweckmäßigerweise so gewählt, dass im gelösten Zustand, d. h. wenn kein weibliches Gegenstück auf den Luer-Konus aufgesetzt ist, aufgrund des Spiels die Gewindehülse weiter um den Luer-Konus gedreht werden kann. Dabei kann aufgrund des Spiels durch radiale Bewegung der Gewindehülse die Gegen-Anlagefläche die Anlagefläche an jeder Stelle passieren. Wie oben beschrieben, sind die Anlagefläche und die Gegen-Anlagefläche jedoch vorzugsweise so ausgestaltet, dass beim Einsetzen des weiblichen Gegenstückes und Verschrauben der Gewindehülse mit deren Gewinde das radiale Spiel eliminiert wird, sodass dann die Anlagefläche und die Gegen-Anlagefläche gegeneinander gepresst werden. Dabei kommt insbesondere der Abschnitt der Gegen-Anlagefläche, welcher radial weiter innen gelegen ist, klemmend an der Anlagefläche zur Anlage.

Dies wird dadurch erreicht, dass die Anlagefläche und die Gegen-Anlagefläche ringförmig, insbesondere kreisringförmig ausgebildet sind und die Anlagefläche und/oder die Gegen-Anlagefläche exzentrisch zu der Längsachse des Luer-Konus angeordnet sind. Dabei kann das Spiel der Gewindehülse so gewählt sein, dass es ohne das eingesetzte weibliche Gegenstück möglich ist, die Gewindehülse so in radialer Richtung relativ zu dem Fuß des Luer-Konus zu verlagern, dass die Achsen der ringförmigen Anlagefläche und der ringförmigen Gegen-Anlagefläche im Wesentlichen deckungsgleich sind, sodass diese relativ zueinander gedreht werden könnten. Dabei wird die Längssachse der Gewindehülse parallel zu der Längsachse des Luer-Konus verschoben. Das radiale Spiel entspricht bevorzugt im Wesentlichen der Exzentrizität öder ist geringfügig kleiner. Durch Einsetzen des weiblichen Gegenstückes wird dann die Längsachse der Gewindehülse mit der Längsachse des Luer-Konus zentriert, sodass die ringförmige Anlagefläche und die ringförmige Gegen-Anlagefläche nicht mehr konzentrisch sondern exzentrisch zueinander angeordnet sind, sodass es in einem Umfangsabschnitt zu einem klemmenden Kontakt von Anlagefläche und Gegen-Anlagefläche kommt. Speziell dieser Umfangsabschnitt insbesondere der Anlagefläche könnte darüber hinaus mit einer Profilierung versehen sein. Vorzugsweise ist die Anlagefläche ringförmig ausgebildet und weist eine Mittel- bzw. Längsachse auf, welche parallel versetzt zu der Längsachse des Luer-Konus ist.

Weiter bevorzugt ist die Gewindehülse am Fuß des Luer-Konus mit Spiel gehalten, welches derart dimensioniert ist, dass es durch den Eingriff des weiblichen Gegenstückes mit dem Luer-Konus und der Gewindehülse eliminiert wird, wodurch dann die Anlagefläche und die Gegen-Anlagefläche miteinander in Eingriff treten. Es kann sich dabei um ein axiales und/oder radiales Spiel bezogen auf die Längsachse des Luer-Konus handeln. Dieses Spiel stellt die freie Drehbarkeit der Gewindehülse sicher, solange das weibliche Gegenstück nicht auf den Luer-Konus aufgesetzt ist. Erst dann wird durch Aufschrauben der Gewindehülse auf das Gewinde des weiblichen Gegenstückes und/oder Einschrauben des weiblichen Gegenstückes in die Gewindehülse das Spiel durch Bewegung der Gewindehülse relativ zum Fuß des Luer-Konus entweder in axialer und/oder radialer Richtung eliminiert, sodass die Anlagefläche und die Gegen-Anlagefläche miteinander in Anlage treten.

Die erfindungsgemäße Anordnung zusätzlicher Klemmmittel kann jedoch nicht nur in dem Fall zum Einsatz kommen, dass eine Drehbarkeit zwischen Gewindehülse und Luer-Lock-Anschluss zum Aufschrauben auf das weibliche Gegenstück erwünscht ist. Es sind auch Luer-Lock-Anschlüsse bekannt, bei welchen die Gewindehülse eigentlich nicht drehbar ausgebildet ist, eine Verdrehung jedoch aufgrund der Materialpaarung zwischen Luer-Konus und Gewindehülse nicht gänzlich verhindert werden kann. Dies kann beispielsweise bei Glasspritzen der Fall sein, bei welchen eine Gewindehülse aus Kunststoff auf einen Luer-Konus bzw. dessen Fuß aus Glas aufgesetzt ist. Auch bei diesen LuerLock-Anschlüssen können die zusätzlichen Klemmmittel zum Einsatz kommen, um dann wenn die Verbindung zwischen weiblichen Gegenstück und dem Luer-Lock-Anschluss mit Luer-Konus hergestellt ist, eine Verdrehung der Gewindehülse zu verhindern. Bei dieser Ausgestaltung wird dann die relative Drehung zwischen Gewindehülse und weiblichen Gegenstück, welche die Klemmung der Klemmmittel verursacht, bevorzugt durch Drehen des weiblichen Gegenstückes in der Gewindehülse erzielt.

Gemäß einer weiteren Ausführungsform der Erfindung weist die Anlagefläche und/oder die Gegen-Anlagefläche eine profilierte Oberfläche auf. Die profilierte bzw. strukturierte Oberfläche kann dazu dienen, die Reibung zwischen Anlagefläche und Gegen-Anlagefläche zu erhöhen. Darüber hinaus können auch beide Flächen derart profiliert sein, dass es zu einem formschlüssigen Eingriff zwischen Anlagefläche und Gegen-Anlagefläche kommt. Beispielsweise könnten Anlagefläche und Gegen-Anlagefläche jeweils gezahnt ausgebildet sein, sodass die Zähne formschlüssig miteinander in Eingriff treten und eine Verdrehsicherung bilden. Eine derartige profilierte, insbesondere gezahnte Struktur kann dabei so ausgebildet werden, dass ein Lösen der Verbindung möglich ist. Dies geschieht dadurch, dass ein erhöhtes Drehmoment auf die Gewindehülse oder das eingeschraubte weibliche Gegenstück aufgebracht wird, durch welches die Reibung zwischen Anlage und Gegen-Anlagefläche überwunden wird. Dabei ist die Reibung bzw. Klemmung der Klemmmittel, d. h. insbesondere in Form einer Anlagefläche und einer Gegen-Anlagefläche so gewählt, dass das Löse-Moment so hoch ist, dass die Verbindung nicht unbeabsichtigt, beispielsweise durch äußere Erschütterungen gelöst werden kann. Durch entsprechende Profilierung bzw. Verzahnung der Anlagefläche und der Gegen-Anlagefläche ist es darüber hinaus möglich die Klemmung so auszugestalten, dass sie nicht zerstörungsfrei reversibel ist. Dazu können beispielsweise Zähne mit einseitig steilen Flanken oder Hinterschneidungen vorgesehen sein, so dass die Zähne als Sperrklinken wirken.

Die Anlagefläche am Fuß des Luer-Konus kann vorzugsweise konisch ausgebildet sein, wobei die konische Anlagefläche sich in entgegengesetzter Richtung zu dem Luer-Konus verjüngt. Das heißt der Luer-Konus erweitert sich in bekannter Weise von seinem freien Ende im Durchmesser zum Fuß des Luer-Konus hin. Am Fuß des Luer-Konus ist dann zusätzlich, beispielsweise im Inneren einer Nut zur Aufnahme eines radialen Vorsprunges der Gewindehülse eine Anlagefläche ausgebildet, welche sich in umgekehrter Richtung verjüngt. Das heißt die Anlagefläche hat den größten Durchmesser an ihrem Ende, welches dem freien Ende des Luer-Konus zugewandt ist. Durch diese Ausgestaltung kann erreicht werden, dass eine Gegen-Anlagefläche bzw. eine Gegen-Anlagekante der Gewindehülse klemmend an der konischen Anlagefläche zur Anlage kommen kann, wenn ein weibliches Gegenstück auf den Luer-Konus aufgesetzt und die Gewindehülse auf das Gewinde des weiblichen Gegenstückes aufgeschraubt bzw. das weibliche Gegenstück in die Gewindehülse eingeschraubt ist. Durch das Aufschrauben der Gewindehülse auf das weibliche Gegenstück bzw. das Einschrauben des weiblichen Gegenstücks wird das weibliche Gegenstück auf den Luer-Konus gezogen bzw. gepresst. Dabei wird gleichzeitig auf die Gewindehülse eine axiale Gegenkraft in Richtung des freien Endes des Luer-Konus ausgeübt. Diese Kraft wird dazu genutzt, die Gegen-Anlagefläche gegen die konische Anlagefläche zu pressen, sodass hier ein zusätzliches Klemmmittel geschaffen wird, welches durch Klemmung bzw. Reibung die Gewindehülse gegen Verdrehung am Fuß des Luer-Konus sicher.

Dazu ist vorzugsweise die Gegen-Anlagefläche korrespondieren konisch zu der Anlagefläche ausgebildet, d. h. die Gegen-Anlagefläche ist bezüglich der Längsachse des Luer-Konus in demselben Winkel geschrägt wie die Anlagefläche. Der Winkel kann ähnlich dem Winkel des Luer-Konus beispielsweise im Bereich von 6° liegen. Durch die korrespondierend konische Ausgestaltung können Anlagefläche und Gegen-Anlagefläche flächig zur Anlage kommen, sodass eine große Reibung zwischen beiden Flächen und damit vorzugsweise eine Selbsthemmung erreicht werden kann.

Weiter bevorzugt sind die Anlagefläche und die Gegen-Anlagefläche konzentrisch zur Längsachse des Luer-Konus angeordnet. Dies ermöglicht es, dass im gelösten Zustand die Gegen-Anlagefläche drehend um die Anlagefläche bzw. relativ zu der Anlagefläche bewegt werdenkann, sodass die Gewindehülse um den Luer-Konus gedreht werden kann.

Neben den vorangehend beschriebenen zwei Beispielen für die Ausgestaltung der Klemmmittel, einmal als konische Anlagefläche, einmal als exzentrisch zueinander angeordnete Anlage- und Gegen-Anlageflächen können alternativ andere Ausgestaltungen, beispielsweise Keilflächen oder ähnliches vorgesehen werden, welche durch Aufschrauben der Gewindehülse auf das weibliche Gegenstück bzw. Einschrauben des weiblichen Gegenstückes in die Gewindehülse klemmend miteinander in Anlage gebracht werden.

Die erfindungsgemäße Ausgestaltung eignet sich insbesondere für Luer-Lock-Anschlüsse, bei welchem der Luer-Konus und die Gewindehülse aus unterschiedlichen Materialien gefertigt sind. Dies kann beispielsweise ein Luer-Konus aus Glas als Teil einer Glasspritze bzw. Kartusche und eine Gewindehülse aus Kunststoff sein. Bei der Anbringung der Gewindehülse aus Kunststoff am Fuß des Luer-Konus aus Glas ist herkömmlich nicht immer eine drehsichere Fixierung möglich. Durch die erfindungsgemäße Ausgestaltung kann diese jedoch zumindest im verbundenen Zustand mit einem Gegenstück sichergestellt werden. Darüber hinaus eignet sich die erfindungsgemäße Ausgestaltung insbesondere auch bei Verwendung von Materialien mit selbstschmierenden Eigenschaften, wie beispielsweise PTFE. Bei solchen Materialien kann durch die zusätzlichen Klemmmittel eine sichere Fixierung der Gewindehülse, wenn diese mit einem Gegenstück verbunden ist, sichergestellt werden.

Nachfolgend wir die Erfindung beispielhaft anhand der beigefügten Figuren beschrieben. In diesen zeigt:
- Fig. 1: eine schematische Schnittansicht eines bekannten Luer-Lock-Anschlusses, welcher kein erfindungsgemäßes Klemmmittel aufweist,
- Fig. 2: eine Vergrößerung des Ausschnitts II in Fig. 1
- Fig. 3: eine perspektivische Schnittansicht einer Variante der Ausführungsform gemäß Fig. 1,
- Fig. 4: eine schematische Ansicht des Luer-Konus gemäß einer Ausführungsform der Erfindung,
- Fig. 5: eine normal zur Längsachse geschnittene Ansicht des Luer-Konus gemäß Fig. 4 mit aufgesetzter Gewindehülse,
- Fig. 6: eine perspektivische Ansicht eines Luer-Konus gemäß einer Variante der Ausführungsform nach Fig. 4 sowie
- Fig. 7: eine normal zur Längsachse geschnittene Ansicht des Luer-Konus gemäß Fig. 6 mit aufgesetzter Gewindehülse.

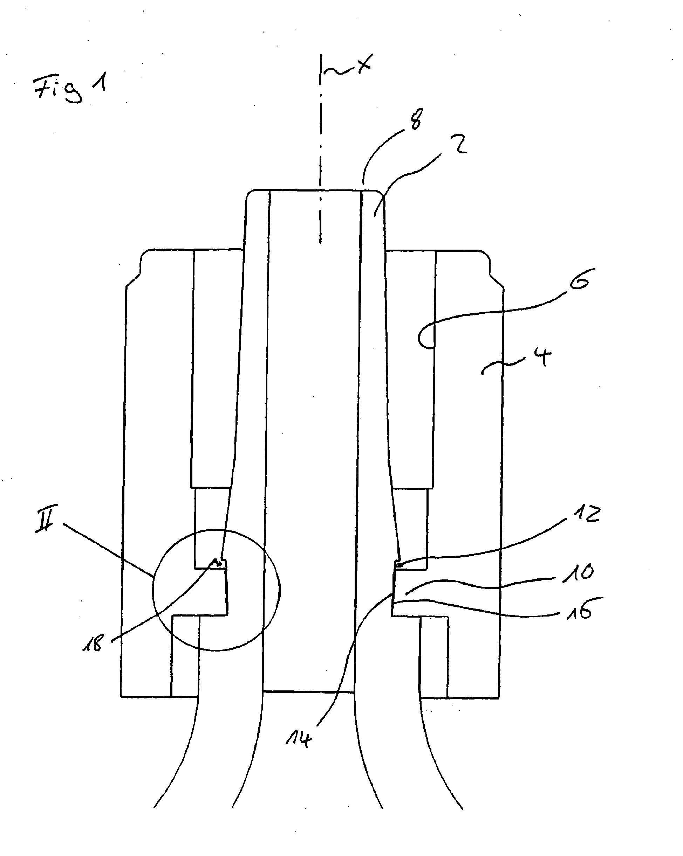

Fig. 1 zeigt einen Luer-Lock-Anschluss mit einem Luer-Konus 2 und einer umgebenden Gewindehülse 4, welche an ihrer Innenumfangsfläche 6 ein hier nicht gezeigtes Innengewinde, wie es für Luer-Lock-Anschlüsse üblich ist, trägt. Die Gewindehülse 4 weist an ihrem dem freien Ende 8 des Luer-Konus 2 abgewandten Ende einen radial nach innen gerichteten ringförmigen Vorspruch 10 auf, welcher in einen ringförmigen Nut 12 am Fuß des Luer-Konus 2 eingreift. Auf diese Weise ist die Gewindehülse 4 in bekannter Weise um die Längsachse X des Luer-Konus 2 relativ zu dem Luer-Konus 2 drehbar, aber in axialer Richtung X gesichert. So kann die Gewindehülse 4 mit ihrem hier nicht gezeigten Innengewinde auf ein korrespondierendes Außengewinde eines weiblichen Gegenstückes, welches auf den Luer-Konus 2 aufgesetzt wird, aufgeschraubt werden, sodass das Gegenstück in Anlage mit dem Luer-Konus 2 gebracht wird.

Es ist nun ein zusätzliches Klemmmittel zur Verdrehsicherung der Gewindehülse 4, wenn diese mit einem hier nicht gezeigten Gegenstück in Eingriff ist, vorgesehen. Dieses Klemmmittel wird von einer Anlagefläche 14 und einer Gegen-Anlagefläche 16 gebildet. Die Anlagefläche 14 wird von dem Boden der Nut 12 gebildet, während die Gegen-Anlagefläche 16 von der Innenumfangsfläche des ringförmigen Vorsprunges 10 an der Gewindehülse 4 gebildet wird. Sowohl die Anlagefläche 14 als auch die Gegen-Anlagefläche 16 sind konisch ausgebildet. Dabei ist der Neigungswinkel so gewählt, dass der Konus sich in entgegengesetzter Richtung zu dem Luer-Konus 2 verjüngt. Das heißt, das im Durchmesser kleinere axialer Ende der Anlagefläche 14 und der Gegen-Anlagefläche 16 ist das dem freien Ende 8 des Luer-Konus 2 abgewandte Ende der Anlagefläche 14 bzw. Gegen-Anlagefläche 16. Der Neigungswinkel der Anlagefläche 14 sowie der Gegen-Anlagefläche 16 kann im Wesentlichen dem Neigungswinkel des Luer-Konus entsprechen.

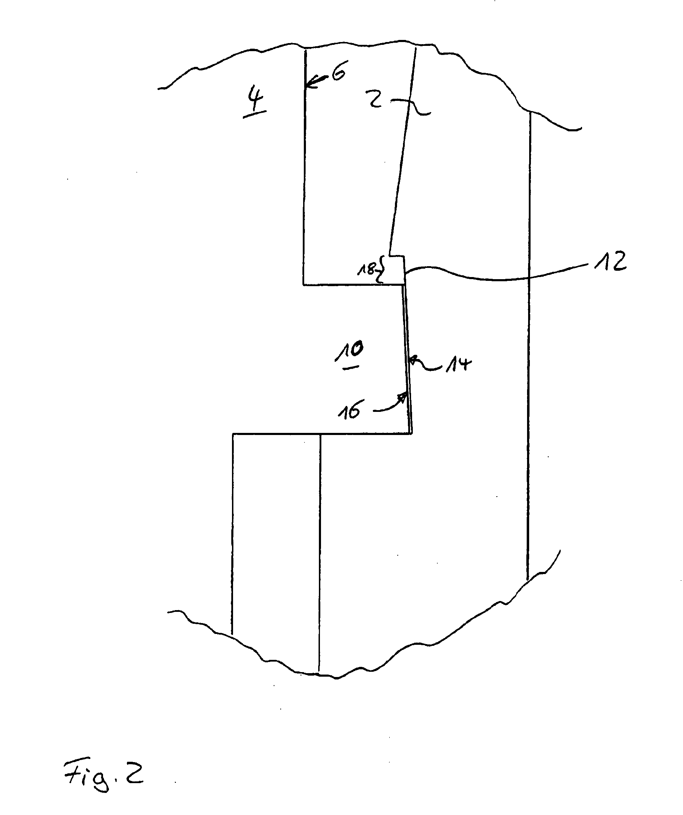

Wie insbesondere in Fig. 2 zu erkennen ist, ist die Nut 12 in axialer Richtung X breiter als der Vorsprung 10, sodass ein axiales Spiel 18 zwischen der Gewindehülse 4 und dem Luer-Konus 2 besteht. Im gelösten Zustand, welcher in Fig. 1 und 2 gezeigt ist, kann die Gewindehülse 4 somit geringfügig vom freien Ende 8 des Luer-Konus 2 nach hinten bewegt werden, sodass die Anlagefläche 14 und die Gegen-Anlagefläche 16 nicht miteinander in klemmender bzw. haftender Anlage sind, wie in Fig. 2 gezeigt. Dies ermöglicht es, die Gewindehülse 4 frei um den Luer-Konus 2 zu drehen. Wenn nun auf den Luer-Konus 2 ein weibliches Gegenstück aufgesetzt wird, mit dessen Außengewinde das Innengewinde der Gewindehülse 4 in Eingriff kommt, wird das weibliche Gegenstück in axialer Richtung auf den Luer-Konus 2 aufgedrückt, wodurch gleichzeitig die Gewindehülse 4 in Richtung des freien Endes 8 des Luer-Konus 2 gedrückt bzw. verlagert wird. Dadurch kommen die Anlagefläche 14 und die Gegen-Anlagefläche 16 miteinander in Anlage, sodass diese gegeneinander gepresst werden. Auf diese Weise wird zusätzliche Reibung bzw. eine zusätzliche Klemmung zwischen der Anlagefläche 14 und der Gegen-Anlagefläche 16 geschaffen, welche eine Drehung der Gewindehülse 4 um die Längsachse X verhindert. Um dennoch die Gewindehülse 4 um die Längsachse X zu drehen, beispielsweise zum Lösen der Verbindung, muss ein erhöhtes Drehmoment aufgebracht werden, um die Reibung zwischen Anlagefläche 14 und Gegen-Anlagefläche 16 zu überwinden. Dieses erhöhte Moment kann so groß sein, dass ein unbeabsichtigtes Lösen, beispielsweise durch Erschütterungen sicher verhindert werden kann. Erfindungsgemäß wird somit eine zusätzliche Klemmung direkt zwischen dem Luer-Konus 2 bzw. dem Fuß des Luer-Konus 2 und der Gewindehülse 4 geschaffen, welche die Gewindehülse 4 im mit einem Gegenstück verbundenen Zustand gegen Drehung sichert.

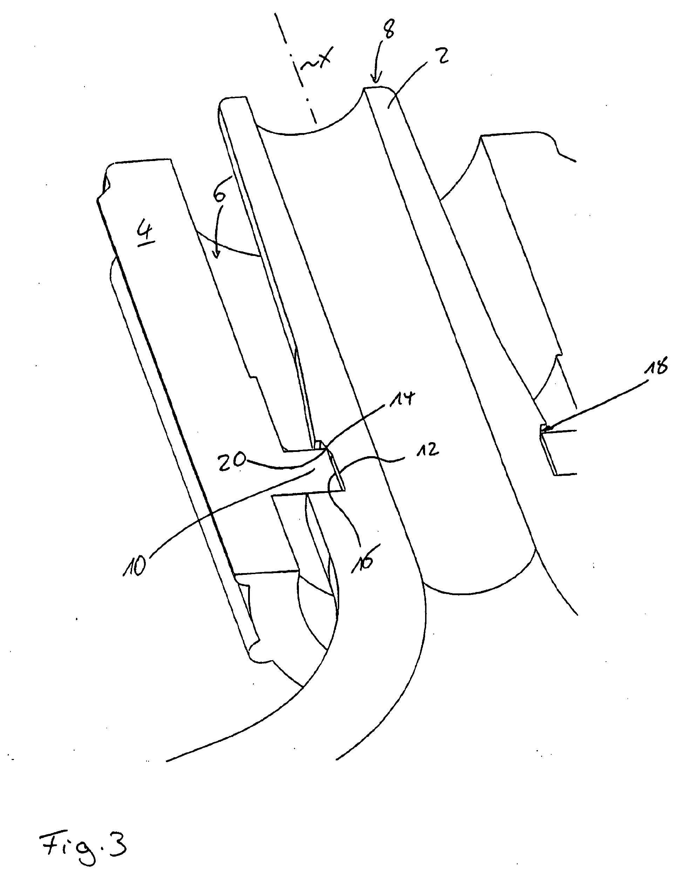

Fig. 3 zeigt eine Variante der Ausführungsform gemäß Fig. 1 und 2. Bei dieser Ausführungsform ist ebenfalls in der Nut 12 eine konische Anlagefläche 14 vorgesehen, jedoch erstreckt diese sich nicht vollständig über die axiale Länge des Nutbodens. Die konische Anlagefläche 14 ist bei dieser Ausführungsform vielmehr nur an dem dem freien Ende 8 des Luer-Konus zugewandten axialen Ende der Nut 12 gelegen. Ferner ist bei dieser Ausführungsform an dem Vorsprung 10 keine korrespondierende Gegen-Anlagefläche 16, welche ebenfalls konisch ist, vorgesehen. Hier wird die Gegen-Anlagefläche vielmehr in Form eine Anlagelinie durch die vordere, d.h. dem freien Ende des Luer-Konus 8 zugewandte Kante 20 des Vorsprunges 10 gebildet. Diese Kante 1 kommt im geklemmten bzw. verbundenen Zustand an der Anlagefläche 14 zur Anlage. Durch den linienförmigen Anlagebereich wird eine hohe Flächenpressung erreicht, sodass auch bei dieser Ausführungsform eine feste Klemmung zwischen der Gewindehülse 4 und dem Luer-Konus 2 zur Verdrehsicherung der Gewindehülse 4 erreicht wird. Im Übrigen entspricht die Ausführungsform gemäß Fig. 3 der vorangehend beschriebenen Ausführungsform.

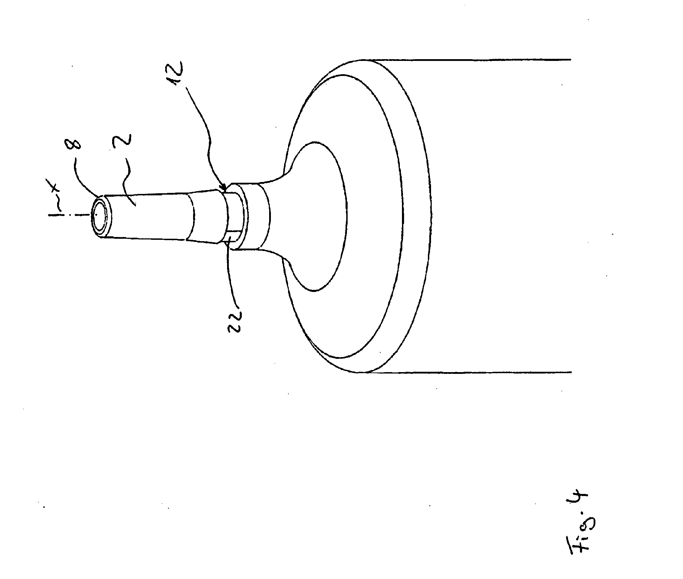

Bei den erfindungsgemäßen Ausführungsformen gemäß Fig. 4 bis 7 wird die Klemmung zwischen der Gewindehülse 4 und dem Luer-Konus 2 nicht, wie bei der vorangehend beschriebenen Ausführungsform in axialer Richtung, sondern in radialer Richtung erzielt.

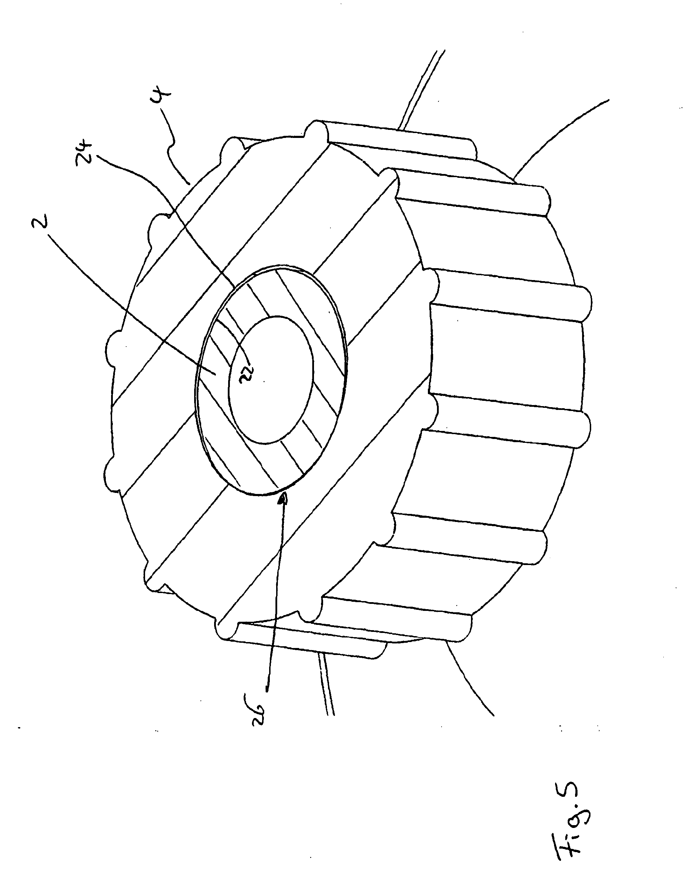

Fig. 4 zeigt den Luer-Konus 2 ohne die aufgesetzte Gewindehülse. Auch bei dieser Ausführungsform ist am Fuß, d. h. am dem freien Ende 8 abgewandten Ende des Luer-Konus 2 eine Nut 12 zur Aufnahme eines radialen Vorsprunges der Gewindehülse vorgesehen. Bei dieser Ausführungsform bildet auch der Nutboden 22 eine Anlagefläche als Teil eines Klemmmittels. Die Anlagefläche 22 ist kreisringförmig ausgebildet, jedoch nicht konzentrisch zur Längsachse X des Luer-Konus 2, sondern exzentrisch, sodass die Längs- bzw. Mittelachse der kreisringförmigen Anlagefläche 22 parallel versetzt zu der Längsachse X des Luer-Konus 2 ist. Wie in der Schnittansicht in Fig. 5 zu erkennen ist, greift die Gewindehülse 4 auch bei dieser Ausführungsform mit einem Vorsprung in die Nut 12 ein. Dabei bildet der Innenumfang des Vorsprungs 12 eine Gegen-Anlagefläche 24. Die Gegen-Anlagefläche 24 ist ebenfalls kreisringförmig ausgebildet, d. h. sie hat die Gestalt eines Kreiszylinders mit einem geringfügigen größeren Durchmesser als die kreiszylindrische Anlagefläche 22. Die Gegen-Anlagefläche 24 ist konzentrisch zu der Innenfläche 6 (siehe Fig. 1 bis 3) der Gewindehülse 4, an welcher das Innengewinde angeordnet ist, ausgebildet. D. h., wenn ein weibliches Gegenstück auf den Luer-Konus 2 aufgesetzt ist, wird die Gewindehülse 4 zu der Längsachse X des Luer-Konus 2 und damit auch die Gegen-Anlageflächen 4 zu dieser Längsachse X zentriert. Da die Anlagefläche 2 jedoch exzentrisch zu der Längsachse X des Luer-Konus 2 ausgebildet ist, kommt es dabei in einem Umfangsabschnitt 26 zur reibschlüssigen bzw. klemmenden Anlage zwischen Anlagefläche 24 und Gegen-Anlagefläche 26. D. h. durch die bei der Verbindung erfolgte Zentrierung der Gewindehülse 4 wird die Gegen-Anlagefläche 26 gegen die Anlagefläche 24 gepresst. Der Umfangsabschnitt 26, in dem es zu dem Kontakt zwischen Anlagefläche 22 und Gegen-Anlagefläche 24 kommt, ist dabei derjenige Umfangsabschnitt, in welchem die Anlagefläche 22 ihren größten radialen Abstand zu der Längsachse X des Luer-Konus hat. Die Dimension des Innendurchmessers der Gegen-Anlagefläche 24 und der Anlagefläche 22 sind dabei so gewählt, dass in der zentrierten Position der Gewindehülse der Radius der Gegen-Anlagefläche 24 idealerweise zumindest geringfügig kleiner ist, als der größte radiale Abstand der Anlagefläche 22 von der Längsachse X des Luer-Konus 2. Auf diese Weise wird eine sichere Klemmung erreicht.

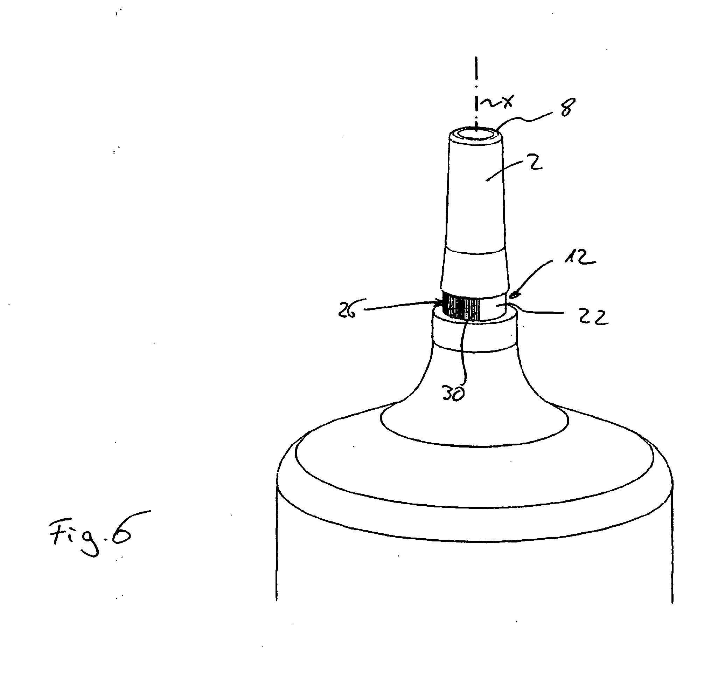

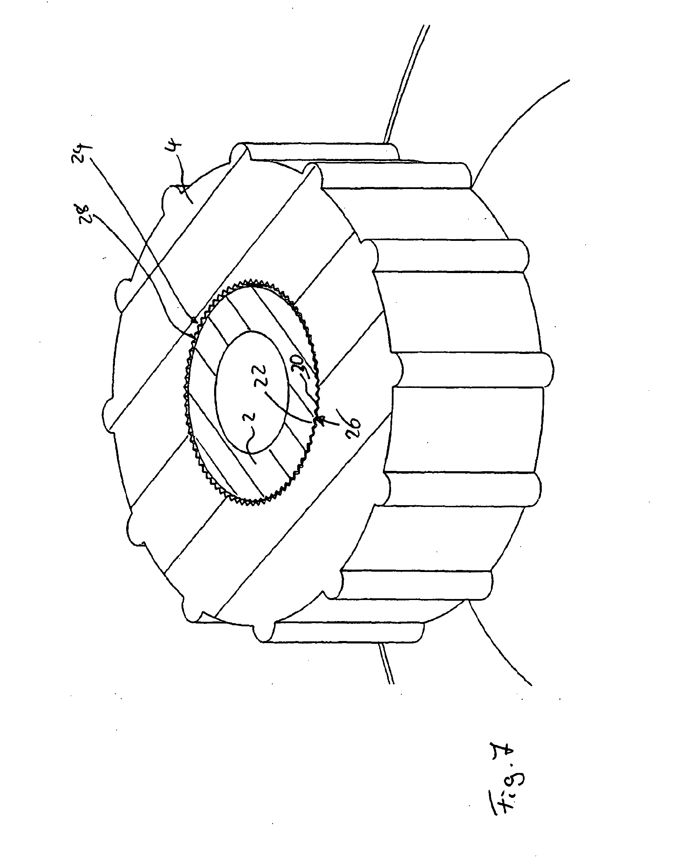

Die Fig. 6 und 7 zeigen eine Variante der Ausführungsform gemäß Fig. 4 und 5, bei welcher zusätzlich eine Profilierung der Anlagefläche 22 und der Gegen-Anlagefläche 24 in Form einer Verzahnung 28, 36 vorgesehen ist. Auf diese Weise wird in dem Umfangsabschnitt 26, in welchem die Anlagefläche 22 zur Anlage an der Gegen-Anlagefläche 24 kommt nicht nur eine reibschlüssige sondern zusätzlich eine formschlüssige Verbindung geschaffen. Die Verzahnung 28 ist über den gesamten Innenumfang der Gegen-Anlagefläche 24 angeordnet. Dahingegen ist eine korrespondierende Verzahnung 30 an der Anlagefläche 22 nur in dem Umfangsabschnitt 26 ausgebildet, in welchen die Anlagefläche 22 und Gegen-Anlagefläche 24 in Eingriff treten. Die Verzahnungen 28 und 30 sind zueinander korrespondierend, d. h. mit im Wesentlichen gleicher Querschnittsform der Zähne ausgebildet, sodass die Verzahnungen 28 und 30 miteinander in Eingriff treten können. Gleichzeitig ist der Innendurchmesser der Gegen-Anlagefläche 24 derart größer gewählt als der Außendurchmesser der Anlagefläche 22, dass die Gegen-Anlagefläche 24 im gelösten Zustand, d. h. wenn die Mittelachse der Gegen-Anlagefläche 24 parallel versetzt zu der Längsachse X des Luer-Konus 2 ist, um die Anlagefläche 22 gedreht werden kann. Auf diese Weise ist sichergestellt, dass im gelösten Zustand die Gewindehülse 4 frei um den Luer-Konus 2 gedreht werden kann.

Es ist zu verstehen, dass die Anlageflächen 14 bzw. 22 sowie Gegen-Anlageflächen 16 und 24 gemäß der vorangehenden Beschreibung auch in anderer Weise profiliert werden können, insbesondere können auch die Anlagefläche 14 und/oder die Gegen-Anlagefläche 16 gemäß der ersten Ausführungsform mit einer Profilierung bzw. Strukturierung zu einem zusätzlichen formschlüssigen Eingriff versehen sein.

Bei den beschriebenen Ausführungsbeispielen ist die Gewindehülse 4 drehbar ausgebildet und das Verschrauben von weiblichem Gegenstück und Gewindehülse 4 erfolgt durch Drehung der Gewindehülse. Es ist jedoch zu verstehen, dass die beschriebene Klemmung zwischen Gewindehülse 4 und dem Fuß des Luer-Konus 2 in identischer Weise erfolgt, wenn nicht die Gewindehülse 4 gedreht wird, sondern das weibliche Gegenstück zum Verschrauben mit der Gewindehülse 4 gedreht wird.

### Bezugszeichenliste

- 2: - Luer-Konus
- 4: - Gewindehülse
- 6: - Innenfläche
- 8: - Freies Ende
- 10: - Vorsprung
- 14: - Anlagefläche
- 16: - Gegen-Anlagefläche
- 18: - Axiales Spiel
- 20: - Kante
- 22: - Anlagefläche
- 24: - Gegen-Anlagefläche
- 26: - Umfangsabschnitt
- 28,30: - Verzahnung
- X: - Längsachse des Luer-Konus

## Patentansprüche

1. Luer-Lock-Anschluss mit einem Luer-Konus (2) und einer diesen umgebenden Gewindehülse (4), bei welchem die Gewindehülse (4) am Fuß des Luer-Konus (2) um dessen Längsachse drehbar angebracht ist,
**dadurch gekennzeichnet, dass**
zwischen der Gewindehülse (4) und dem Fuß des Luer-Konus (2) Klemmmittel (22,24) vorgesehen sind, welche zumindest eine Anlagefläche (14;22) am Fuß des Luer-Konus (2) und eine bezüglich der Längsachse (X) des Luer-Konus (2) radial gegenüberliegende Gegen-Anlagefläche (16;24) an der Gewindehülse (4) aufweisen, die Anlagefläche (22) und die Gegen-Anlagefläche (24) ringförmig, insbesondere kreisringförmig ausgebildet sind und die Anlagefläche (22) und/oder die Gegen-Anlagefläche (24) exzentrisch zu der Längsachse (X) des Luer-Konus (2) angeordnet ist, die Gewindehülse am Fuß des Luer-Konus (2) mit radialen Spiel angeordnet ist und die Anlagefläche (22) und die Gegenanlagefläche (24) derart angeordnet sind, dass sie bei auf den Luer-Konus (2) aufgesetztem weiblichen Gegenstück durch Drehung der Gewindehülse (4) oder Drehung des weiblichen Gegenstückes in der Gewindehülse (4) miteinander in Eingriff treten

2. Luer-Lock-Anschluss nach Anspruch 1, **dadurch gekennzeichnet, dass** die Klemmmittel derart ausgestaltet sind, dass sie durch Drehung der Gewindehülse (4) oder des weiblichen Gegenstückes in der Gewindehülse (4) eine Reib- und/oder Formschlussverbindung zwischen der Gewindehülse und dem Fuß des Luer-Konus bewirken.

3. Luer-Lock-Anschluss nach Anspruch 1 oder 2, **dadurch gekennzeichnet, dass** die Gewindehülse (4) am Fuß des Luer-Konus (2) mit Spiel gehalten ist, welches derart dimensioniert ist, dass es durch den Eingriff des weiblichen Gegenstücks mit dem Luer-Konus (2) und der Gewindehülse (4) eliminiert wird, wodurch die Anlagefläche (22) und die Gegen-Anlagefläche (24) miteinander in Eingriff treten.

4. Luer-Lock-Anschluss nach einem der vorangehenden Ansprüche, **dadurch gekennzeichnet, dass** die Anlagefläche (22) und/oder die Gegen-Anlagefläche (24) eine profilierte Oberfläche (28,30) aufweisen.

5. Luer-Lock-Anschluss, nach einem der vorangehenden Ansprüche, **dadurch gekennzeichnet, dass** die Anlagefläche (14) am Fuß des Luer-Konus (2) konisch ausgebildet ist, wobei die konische Anlagefläche (14) sich in entgegengesetzter Richtung zu dem Luer-Konus (2) verjüngt.

6. Luer-Lock-Anschluss nach Anspruch 5, **dadurch gekennzeichnet, dass** die Gegen-Anlagefläche (16) korrespondierend konisch zu der Anlagefläche (14) ausgebildet sind.

7. Luer-Lock-Anschluss nach Anspruch 5 oder 6, **dadurch gekennzeichnet, dass** die Anlagefläche (14) und die Gegen-Anlagefläche (16) konzentrisch zur Längsachse des Luer-Konus (2) angeordnet sind.

8. Luer-Lock-Anschluss, nach einem der vorangehenden Ansprüche, **dadurch gekennzeichnet, dass** der Luer-Konus (2) und die Gewindehülse (4) aus unterschiedlichen Materialien gefertigt sind.

## Claims

1. A Luer lock connection with a Luer cone (2) and with a threaded sleeve (4) surrounding this, with which the threaded sleeve (4) is attached on the foot of the Luer cone (2) in a rotatable manner about the longitudinal axis of said Luer cone,
**characterised in that**
clamping means (22, 24) are provided between the threaded sleeve (4) and the foot of the Luer cone (2), comprising at least one contact surface (14; 22) on the foot of the Luer cone (2) and a counter-contact surface (16; 24) on the threaded sleeve (4) lying radially opposite with respect to the longitudinal axis (X) of the Luer cone (2), the contact surface (22) and the counter-contract surface (24) are designed in an annular manner, in particular in an annulus-shaped manner, and the contact surface (22) and/or the counter-contact surface (24) is arranged eccentrically to the longitudinal axis (X) of the Luer cone (2), the threaded sleeve is arranged on the foot of the Luer cone (2) with radial play and the contact surface (22) and the counter-contact surface (24) are arranged in a manner such that with a female counter-piece put onto the Luer cone (2), they come into engagement with one another by way of rotation of the threaded sleeve (4) or rotation of the female counter-piece in the threaded sleeve (4)

2. A Luer lock connection according to claim 1, **characterised in that** the clamping means are designed in a manner such that they effect a friction-fit connection and/or positive-fit connection between the threaded sleeve and the foot of the Luer cone, by way of rotation of the threaded sleeve (4) or of the female counter-piece in the threaded sleeve (4).

3. A Luer lock connection according to claim 1 or 2, **characterised in that** the threaded sleeve (4) is held on the foot of the Luer cone (2) with play which is dimensioned in a manner such that it is eliminated by the engagement of the female counter-piece with the Luer cone (2) and the threaded sleeve (4), by which means the contact surface (22) and the counter-contact surface (24) come into engagement with one another.

4. A Luer lock connection according to one of the preceding claims, **characterised in that** the contact surface (22) and/or the counter-contact surface (24) have a profiled surface (28; 30).

5. A Luer lock connection according to one of the preceding claims, **characterised in that** the contact surface (14) is designed in a conical manner on the foot of the Luer cone (2), wherein the conical contact surface (14) tapers in an opposite direction to the Luer cone (2).

6. A Luer lock connection according to claim 5, **characterised in that** the counter-contact surface (16) is designed conically to the contact surface (14) in a corresponding manner.

7. A Luer lock connection according to claim 5 or 6, **characterised in that** the contact surface (14) and the counter-contact surface (16) are arranged concentrically to the longitudinal axis of the Luer cone (2).

8. A Luer lock connection according to one of the preceding claims, **characterised in that** the Luer cone (2) and the threaded sleeve (4) are manufactured of different materials.

## Revendications

1. Raccord luer-lock comprenant un cône de luer-lock (2) et un manchon
fileté (4) qui entoure ce cône, le manchon fileté (4) étant disposé de manière à pouvoir tourner au niveau du pied du cône de luer-lock (2) autour de l'axe longitudinal de ce cône,
**caractérisé en ce qu'**
entre le manchon fileté (4) et le pied du cône de luer (2), sont prévus des moyens de coincement (22, 24) qui présentent au moins une face de portée (14 ; 22) formée sur le pied du cône de luer (2) et une face de portée conjuguée (16 ; 24) formée sur le manchon fileté (4), en face dans la direction radiale, relativement à l'axe longitudinal (X) du cône de luer (2), la face de portée (22) et la face de portée conjuguée (24) sont de forme annulaire, en particulier de forme annulaire circulaire, et la face de portée (22) et/ou la face de portée conjuguée (24) est ou sont disposée(s) excentriquement par rapport à l'axe longitudinal (X) du cône de luer (2), le manchon fileté étant disposé avec un jeu radial au niveau du pied du cône de luer (2), et la face de portée (22) et la face de portée conjuguée (24) étant disposées de telle sorte que, lorsqu'une pièce conjuguée femelle est posée sur le cône de luer (2), elles entrent en prise entre elles sous l'effet de la rotation du manchon fileté (4) ou sous l'effet de la rotation de la pièce conjuguée femelle dans le manchon fileté (4).

2. Raccord luer-lock selon la revendication 1, **caractérisé en ce que** les moyens de coincement sont conformés de telle sorte, sous l'effet de la rotation du manchon fileté (4) ou sous l'effet de la rotation de la pièce conjuguée femelle dans le manchon fileté (4), ils établissent une liaison par frottement et/ou par complémentarité de forme entre le manchon fileté et le pied du cône de luer.

3. Raccord luer-lock selon la revendication 1 ou 2, **caractérisé en ce que** le manchon fileté (4) est tenu au niveau du pied du cône de luer (2) avec un jeu qui est d'une largeur telle qu'il est éliminé par l'entrée en prise de la pièce conjuguée femelle avec le cône de luer (2) et avec le manchon fileté (4), de sorte que la face de portée (22) et la face de portée conjuguée (24) entrent en prise entre elles.

4. Raccord luer-lock selon une des revendications précédentes, **caractérisé en ce que** la face de portée (22) et/ou la face de portée conjuguée (24) présente(nt) une surface profilée (28, 30).

5. Raccord luer-lock selon une des revendications précédentes, **caractérisé en ce que** la face de portée (14) est conique au niveau du pied du cône de luer (2), la face de portée conique (14) se rétrécissant en sens inverse du cône de luer (2).

6. Raccord luer-lock selon la revendication 5, **caractérisé en ce que** la face de portée conjuguée (16) est d'une forme conique qui correspond à la face de portée (14).

7. Raccord luer-lock selon la revendication 5 ou 6, **caractérisé en ce que** la face de portée (14) et la face de portée conjuguée (16) sont disposées concentriquement à l'axe longitudinal du cône de luer-lock (2).

8. Raccord luer-lock **caractérisé en ce que** le cône de luer (2) et le manchon fileté (4) sont fabriqués en des métaux différents.
